# EUROPÄISCHE PATENTSCHRIFT

(11) **EP 1 405 632 B1**
(45) Veröffentlichungstag und Bekanntmachung des Hinweises auf die Patenterteilung: **18.08.2010**
(21) Anmeldenummer: 03022236.8
(22) Anmeldetag: 01.10.2003
(51) Int. Cl.: A61K 8/31, A61K 8/27, A61K 8/02

(54) **Imprägnierte kosmetische Tücher, welche nanoskalige Zinkoxide enthalten**
Impregnated cosmetic tissues comprising nanoscale zinc oxide
Serviette cosmétique imprégnée d'une composition contenant des nano-oxydes de zinc

(30) Priorität: 02.10.2002 DE 10246160
(43) Veröffentlichungstag der Anmeldung: 07.04.2004
(73) Patentinhaber: Beiersdorf AG, 20245 Hamburg (DE)
(72) Erfinder: Thaden von, Stefanie, 20255 Hamburg (DE); Schäfer, Andreas, Dr., 22529 Hamburg (DE); Hahn, Ingo, Dr., 25421 Pinneberg (DE); Fecht von der, Stephanie, 22869 Schenefeld (DE); Koopmann, Sabine, 20253 Hamburg (DE)

(56) Entgegenhaltungen:
- WO-A-00/54733
- WO-A-00/78281
- WO-A-02/49684
- WO-A-97/23247
- WO-A-99/59540
- US-A1- 2002 058 754
- DATABASE WPI Section Ch, Week 199805 Derwent Publications Ltd., London, GB; Class A14, AN 1998-042584 XP002266418 & AU 22216 97 A (UNILEVER PLC), 27. November 1997 (1997-11-27)

## Beschreibung

Die Erfindung betrifft flexible und saugfähige Substrate, die mit kosmetischen oder dermatologischen Wirkstoffen oder Zubereitungen in Form von sehr feinteiligen Partikeln im Nanometerbereich ausgerüstet sind und sich zur reinigenden, pflegenden oder therapeutischen Behandlung der Haut eignen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Die Haut ist das größte Organ des Menschen. Unter ihren vielen Funktionen (beispielsweise zur Wärmeregulation und als Sinnesorgan) ist die Barrierefunktion, die wohl wichtigste. Die Haut wirkt dabei als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden.

Kosmetische und/oder dermatologische Reinigungspräparate werden in aller Regel in Form eines Schaums mit Wasser auf die zu reinigenden Körperpartien aufgetragen. Basis fast PAWI\2002\201-130.doc aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Die meisten kosmetischen und dermatologischen Reinigungszubereitungen liegen in Form von mehr oder weniger viskosen Flüssigkeiten (Reinigungswässer und -milche) oder als Feststoff (z.B. Seife, Waschsynthete) vor. Sie werden in der Regel mit den Händen oder Fingern auf die Haut aufgetragen

Eine besondere Produktform für Reinigungszubereitungen stellen feste Reinigungssubstrate, insbesondere Tücher dar. Diese können bereits vom Hersteller mit der Reinigungszubereitung getränkt sein und haben dadurch den Vorteil, daß in ihnen die Zubereitung bereits in der richtigen Dosierung vorgegeben ist. Außerdem vermeiden sie den Nachteil von in Flaschen aufbewahrten Zubereitungen, deren Verpackung zerbrechen und deren Inhalt "auslaufen" kann. Zu den weiteren Vorteilen von Reinigungssubstraten/Tüchern zählen auch die Umstände, daß sie sich bequem in abgezählter Menge mit auf Reisen nehmen lassen und für ihre Anwendung in der Regel kein Wasser mehr erforderlich ist.

Reinigungssubstrate/Tücher werden aus Textilien hergestellt. Textilien können gewebt, gestrickt oder gewirkt sein oder als Verbundstoff (engl. nonwoven textile) vorliegen. Meist werden (aus Kostengründen) Verbundstoffe verwendet. Bei Verbundstoffen erfolgt die Gewebebildung nicht durch Kette und Schuss oder Maschenbildung, sondern durch Verschlingung, und/oder kohäsive und/oder adhäsive Verbindung von Textilfasern. Verbundstoffe können nach der DIN 61210 T2 in Vlies, Papier Watte und Filz unterschieden werden. Vliese sind lockere Materialien aus Spinnfasern (d.h. Faser mit begrenzter Länge) oder Filamenten (Endlosfasern), meist aus Polypropylen, Polyester oder. Viskose hergestellt, deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. AcrylatPolymere, SBR/NBR, Polyvinylester, Polyurethan-Dispersionen) oder durch Schmelzen oder Auflösen von sogenannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweißt [J. Falbe, M. Regnitz: Römpp-Chemie-Lexikon, 9. Aufl. Thieme-Verlag, Stuttgart (1992)].

Mit kosmetischen Zubereitungen imprägnierte Substrate und insbesondere Tücher können auf unterschiedlichen Wegen hergestellt werden: Im sogenannten "Tauch-Verfahren" wird das Tuch in einem Tauchbad eingetaucht oder durch ein Bad gezogen. Dieses Verfahren eignet sich insbesondere für Papiertücher und weniger für Vliesse, da letztere zu viel Flüssigkeit (=Zubereitung) aufnehmen und sich in Umverpackung anschließend Pfützen von wieder freigesetzter Zubereitung finden.

Eine zweite Variante stellt das "Sprüh-Verfahren" dar, bei dem die Zubereitung auf das vorbeilaufende Tuch aufgesprüht wird. Diese Verfahren eignet sich für alle Textilien, doch können keine stark schäumenden Zubereitungen auf das Tuch aufgebracht werden, da die Schaumentwicklung beim Sprühverfahren zu groß wird.

Als weitere Methode kommen sogenannte Abstreifmethoden zum Einsatz. Dort laufen Vlies oder Tuchbahnen an Abstreifblechen, -balken oder -düsen vorbei, die kontinuierlich mit Imprägnierungslösung beladen werden. Unterschiedliche lmprägnierungsgrade lassen sich u. a. durch Variation des Anpressdruckes und der Tuchzuggeschwindigkeit einstellen.

Nach dem Stande der Technik ist es bisher nur unzureichend möglich Gleitmittel auf Reinigungssubstrate, insbesondere auf Vliese aufzubringen. Gleitmittel (beispielsweise Talkum, Bornitrid oder Stärke (-derivate)), auch Fliesmittel genannt, gehören zu den Puderrohstoffen und sind für die Herstellung von pulverförmigen Zubereitungen geeignete, pulverförmige, meist sehr feinteilige Stoffe, die je nach dem Verwendungszweck über ein entsprechendes Feuchtigkeits- oder Fettaufnahme-, Gleit-, Deck- u. Haftvermögen verfügen. In flüssigen Zubereitungen, beispielsweise in Tränkbädern, neigen Gleitmittel dazu, sich auf dem Boden des Bades abzusetzen und damit zu einer ungleichmäßen Imprägnierung des Tuches mit Gleitmittel im Tauchbadverfahren zu führen. Beim Sprühverfahren führen die Gleitmittel als Feststoffe häufig zur Verstopfung der Sprühdüsen.

Es war daher die Aufgabe der vorliegenden Erfindung, Substrate zu entwickeln, welche die Nachteile des Standes der Technik mindern beziehungsweise umgehen.

Überraschend gelöst wird die Aufgabe durch kosmetische Produkte, umfassend ein Substrat, welches imprägniert ist mit einer kosmetischen und/oder dermatologischen Zubereitung enthaltend
a) Wasser
b) Öl
c) einen oder mehrere Emulgatoren
d) Zinkoxid einer Partikelgröße, die aus dem Bereich von 20 bis 100 nm gewählt wird.

Die erfindungsgemäßen Zubereitungen verleihen der Haut nach der Anwendung ein überraschend angenehmes, samtiges Hautgefühl.

Zwar beschreibt die WO 00/54733 flexible und saugfähige mit kosmetischen oder dermatologischen Wirkstoffen oder Zubereitungen ausgerüstete Träger zur Behandlung der Haut, dadurch gekennzeichnet, dass als Ausrüstung Wirkstoffe oder Zubereitungen, die bei 40 °C fest sind, in Form von Nanopartikeln mit einem mittleren Teilchendurchmesser von weniger als 300 nm darin enthalten sind., doch konnte diese Schrift nicht den Weg zur vorliegenden Erfindung weisen.

Weiterer Stand der Technik sind WO 0 249 684, WO 995 940, US 6 004 567, US 5 643 557 und WO 9 803 713.

In überraschender Weise hat sich nämlich gezeigt, daß bei der Wahl einer Partikelgröße die außerhalb des erfindungsgemäßen Bereiches von 20 bis 100 nm liegt, keine stabilen kosmetischen Produkte erhältlich sind, sondern daß vielmehr ein unschönes "Weißeln" zu vermerken ist.

Unter imprägnierten Substraten werden erfindungsgemäß Substrate verstanden, die durch Ansprühen, Eintauchen oder Abstreifen mit der kosmetischen Zubereitung getränkt wurden. Der Begriff imprägniert umfasst sowohl sich feucht anfühlende, frisch getränkte Substrate als auch durch einen Trocknungsprozess getrocknete bzw. sich trocken anfühlende Substrate, welche die kosmetische Zubereitung in konzentrierter Form enthalten.

Die erfindungsgemäßen Substrate können glatt oder auch oberflächenstrukturiert sein. Erfindungsgemäß bevorzugt sind oberflächenstrukturierte Substrate.

Bei den erfindungsgemäßen Substraten kann die Gewebebildung durch Kette und Schuss, durch Maschenbildung oder durch Verschlingung, und/oder kohäsive und/oder adhäsive Verbindung von Textilfasern erfolgen. Dabei ist es erfindungsgemäß bevorzugt, wenn es sich bei dem Substrat um ein Verbundstoff handelt.

Erfindungsgemäß bevorzugt werden Substrate in Form von Tüchern eingesetzt, welche aus Vlies bestehen, insbesondere aus wasserstrahlverfestigten und/oder wasserstrahlgeprägten Vlies. Die Substrate können vorteilhaft auch als Bausch, gelochtes Vlies oder Netz ausgeführt sein.

Derartige Substrate können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 20 bis 120 g/m², vorzugsweise von 30 bis 80 g/m² besonders bevorzugt 40 bis 60 g/m² hat (gemessen bei 20 °C ± 2 °C und bei einer Feuchtigkeit der Raumluft von 65 % ± 5 % für 24 Stunden).

Die Dicke des Substrates beträgt vorzugsweise 0,2 mm bis 2 mm, insbesondere 0,4 mm bis 1,5 mm, ganz besonders bevorzugt 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute; Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer vorteilhaften Ausführungsform des Vlieses bestehen die Fasern aus einer Mischung aus 60 % bis 80 %Viskose mit 40% bis 20 % PET, insbesondere 70% Viskose und 30 % PET. Besonders vorteilhaft ist eine Mischung aus 70 %Viskose und 30 % PET.

Besonders vorteilhaft sind auch Fasern aus hochfesten Polymeren wie Polyamid, Polyester und/oder hochgerecktem Polyethylen.

Es ist erfindungsgemäß vorteilhaft, wenn das erfindungsgemäße Tuch aus einem Faservlies besteht, welches durch ein wasserunlösliches Bindemittel zusammengehalten wird.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Ferner weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt |
| | | 20 % und 50 % |
| | Querrichtung | 40 % bis 120 %, bevorzugt |
| | | 50 % und 85 % |
| im getränkten Zustand | Maschinenrichtung | 15 % bis 100 %, bevorzugt |
| | | 20 % und 40 % |
| | Querrichtung | 40 % bis 120 %, bevorzugt |
| | | 50% und 85% |

Es ist erfindungsgemäß vorteilhaft, wenn die Zubereitung zum Zeitpunkt des Auftragens auf das Substrat das Zinkoxid in einer Gesamtkonzentration von 0,01 bis 20 Gewichts-%, bevorzugt in einer Gesamtkonzentration von 0.05 bis 10 Gewichts-% und ganz besonders bevorzugt in einer Gesamtkonzentration von 0.1 bis 5 Gewichts-%, jeweils bezogen auf das Gesamtgewicht der Zubereitung enthält.

Auch ist es erfindungsgemäß von Vorteil, wenn die Gesamtkonzentration an Zinkoxid von 0,01 bis 5 mg pro cm² Substrat, bevorzugt von 0,05 bis 1 mg pro cm² und ganz besonders bevorzugt von 0,1 bis 0,5 mg pro cm² beträgt.

Eine Vermeidung der bei niedrigviskosen (Vis<500 mPas, Harke VT03) kosmetischen Formulierungen zu beobachtenden Sedimentation von partikulären Füllstoffen, die eine höhere Dichte als das sie umgebende Medium besitzen (typischerweise : Dichte >2), kann durch den Einsatz nanoskaliger Partikel (Durchmesser < 200 nm) erreicht werden. Somit können lagerstabile homogene Suspensionen, insbesondere von Metalloxiden wie ZnO, TiO₂ ZrO₂, CeO₂, SnO₂, Al₂O₃ in niedrigviskosen wssrg. Systemen, wie wssrg. Lösungen, O/W Emulsionen (insbesondere PIT-Emulsionen, Mikroemulsionen), W/O-Emulsionen, multiplen Emulsionen (z.B. W/O/W) und Hydrodispersionen (Hydrogele) erhalten werden.

Geeignete Zinkoxide sind bespielsweise die von der Firma ANT-Powders durch das mechanochemische MCP-Verfahren, wie in WO 9959754 beschrieben oder auch durch das in EP 854765 beschriebene Verfahren, erhältichen Nanoteilchen. Für den Einsatz in Tränkungsmedien sind v.a. die nanopartikulären Zinkoxide von Bedeutung, die von o.g. Firma unter dem Handelsnamen zinClear® angeboten werden. Die Partikel besitzen eine typische Durchschnittsgröße zwischen 25 und 100 nm und werden typischerwiese als Suspension in wässrigen oder lösungsmittelbasierten Systemen wie z.B. in Capric / Caprylic Triglycerides, Iso-Propylpalmitat oder Finsolv TN angeboten. Weiterhin sind z.B. die Partikel der Firma Elementis, die unter dem Handelsnamen Nanox vertrieben werden geeignet.

Die mit diesem Pigment erhältlichen Systeme sind insbesondere als Tränkungsmedium für kosmetische Reinigungs-, Pflege-, Baby-, Deo- und Sonnenschutztücher geeignet.

In erstaunlicher Weise zeichnen sich dermaßen ausgestaltete kosmetische Produkte durch hervorragende UV-Schutzleistung sowie insbesondere bakterizide und fungizide Wirkung aus.

Produkte entsprechend der vorliegenden Erfindung enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; lsosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-lsostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/C₁₀₋₃₀-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; lsosteareth-10; Polyglyceryl-2-sesquiisostearat; Ceteth-10; Polyglyceryl-2 Dipolyhydroxystearat; Oleth-20; lsoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, lsopropylstearat, lsopropyloleat, n-Butylstearat, n-Hexyllaurat, n-Decyloleat, lsooctylstearat, lsononylstearat, lsononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅ -Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und lsotridecylisononanoat, aus Cyclomethicon und 2-Ethylhexylisostearat.

Die erfindungsgemäßen Produkte können erfindungsgemäß vorteilhaft anionische Tenside, kationische Tenside, amphotere Tenside, nichtionische Tenside und/oder Polysorbate enthalten.

Es ist besonders vorteilhaft das oder die erfindungsgemäßen waschaktiven Tenside aus der Gruppe der Tenside zu wählen, welche einen HLB-Wert von mehr als 15 haben, ganz besonders vorteilhaft sind solche, welchen einen HLB-Wert von mehr als 25 haben.

Besonders vorteilhafte waschaktive anionische Tenside im Sinne der vorliegenden Erfindung sind beispielsweise
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acyl-isethionate, z.B. Natrium-/ Ammoniumcocoyl-isethionat,
■ Olefinsulfonate,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Sulfosuccinatcitrate. sowie Schwefelsäureester, wie
■ Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quarternäre Tenside. Quaternäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amidopropylhydroxysultain.

Vorteilhafte waschaktive amphotere Tenside im Sinne der vorliegenden Erfindung sind beispielsweise
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Besonders vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, insbesondere Monoalkylphosphate, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitöylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist erfindungsgemäß vorteilhaft, kristallisierbare anionische, kationische, amphotere, nichtionische Tenside in den erfindungsgemäßen Zubereitungen einzusetzen.

Es ist erfindungsgemäß bevorzugt, als Tenside eine Kombination aus einem oder mehreren anionischen Tensiden und einem oder mehreren nichtionischen Tensiden einzusetzen.

Hierbei werden bevorzugt ein oder mehrere anionische Tenside gewählt aus der Gruppe Acylaminosäuretenside, Sarkosinate, Sulfosuccinate, Sulfosuccinatcitrate, Monoalkylphosphate, Olefinsulfonate.

Als nichtionische Tenside werden bevorzugt ein oder mehrere Alkylpolyglucoside eingesetzt, wobei Laurylglucosid und/oder Decylglucosid die erfindungsgemäß besonders bevorzugten nichtionischen Tenside darstellen.

Erfindungsgemäß ganz besonders bevorzugt ist eine Tensidkombination aus Natriumcocoylglutamat und Laurylglucosid und/oder Decylglucosid.

Vorteilhaft im Sinne der vorliegenden Erfindung beträgt das Verhältnis von anionischen zu nichtionischen Tensiden: 2:5 bis 5:2, Bevorzugte Verhältnisse sind dabei 5:3, 3,5:4 und insbesonders 2,5:3 (jeweils anionische zu nichtionische Tenside).

Vorteilhaft im Sinne der vorliegenden Erfindung enthält die Zubereitung zum Zeitpunkt des Auftragens auf das Substrat ein oder mehrere Tenside in einer Gesamtkonzentration von 5 bis 90 Gewichts-%, bevorzugt von 40 bis 85 Gewichts-% und ganz besonders bevorzugt von 55 bis 80 Gewichts-%, jeweils bezogen auf das Gesamtgewicht der Zubereitung.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol; Imidazolidinylharnstoff, 5-Chlor-2-methyl-4-isothiazolin-3-on, 2-Chloracetamid, Benzalkoniumchlorid, Benzylalkohol.

Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung in der Zusammensetztung welche diese zum Zeitpunkt des Auftragens auf das Substrat aufweist, in der erfindungsgemäßen Zubereitung enthalten.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im *Intemational Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen.

Erfindungsgemäß vorteilhafte öllösliche Konditionierer sind beispielsweise Mineralöl, Paraffinöl, Vasiline (Petrolatum), C-7 bis C-40 Kohlenwasserstoffe (gesättigt und ungesättigt sowie linear und verzweigt, z.B. Squalan und Squalen, Polyolefine, hydrogenierte Polyisobutene und Isohexadecan), C-1 bis C-30 Alkohole (gesättigt und ungesättigt sowie linear und verzweigt), Ester von C-1 bis C-30 Alkoholen (gesättigt und ungesättigt sowie linear und verzweigt) mit C-1 bis C-30 Mono- und Dicarbonsäuren (gesättigt und ungesättigt sowie linear und verzweigt, beispielsweise Octylpalmitat, Octylcocoat, Octylisostearat, Octyldodeceylmyristat, Octyldodekanol, Cetearylisononanoat, Isopropylmyristat, lsopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl). Auch Mono-, Di- und Triglyceride von C-1 bis C-30 Carbonsäuren (gesättigt und ungesättigt sowie linear und verzweigt), Ethylenglycolmono- und Diester von C-1 bis C-30 Carbonsäuren (gesättigt und ungesättigt sowie linear und verzweigt), Propylenglycolmono- und Diester von C-1 bis C-30 Carbonsäuren (gesättigt und ungesättigt sowie linear und verzweigt), Mono- und Polyester von Kohlenhydraten (insbesondere Zucker) und C-1 bis C-30 Carbonsäuren (gesättigt und ungesättigt sowie linear und verzweigt), Lecithine, Pflanzenöle (wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Jojobaöl), hydrierte Pflanzenöle, Polypropylenglycolether und Polyethylenglycolether von Pflanzenölen, hydrierten Pflanzenölen und Alkylkohlenwasserstoffen gehören zu den erfindungsgemäß vorteilhaften Konditionierern. Zu den erfindungsgemäß vorteilhaften öllöslichen Konditionierer zählen außerdem die natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner können die Konditionierer vorteilhaft gewählt werden aus der Gruppe der Dialkylether und Dialkylcarbonate, vorteilhaft sind z. B. Dicaprylylether *(Cetiol OE*) und/oder Dicaprylylcarbonat, beispielsweise das unter der Handelsbezeichnung Cetiol CC bei der Fa. Cognis erhältliche.

Weitere vorteilhafte Konditionierer können auch aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn als öllösliche Konditionierer ein oder mehrere C₁₂₋₁₅-Alkylbenzoate eingesetzt werden.

Vorteilhafte Konditionierer sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon *(Hallstar AB)* und/oder Diethylhexylnaphthalat (*Hall brite TQ von CP Hall oder Corapan®TQvon Haarmann & Reimer).*

Zu den vorteilhaften öllöslichen Konditionierern zählen auch alle aliphatischen und aromatischen und Silikonöle (Polysiloxane). Die Silikonöle können linear, verzweigt, netzartig oder cyclisch (Cyclomethicone) aufgebaut sein. Als Kohlenwasserstoffreste können beispielsweise Methyl- Ethyl-, Propyl-, Phenyl-Gruppen an die Siliziumatome gebunden sein.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Als wasserlösliche Konditionierer können Alkohole, Polyole (insbesondere Diole und Triole), Polyethylenglycole (PEG-2 bis PEG-200), Polypropylenglycole (PPG-2 bis PPG-200), Hamstoff und seine Derivate, Pyrolidoncarbonsäuren, ethoxylierte und propyloxylierte Di- und Triole sowie α-Hydroxysäuren, Kohlenyhdrate (insbesondere Zucker) ethoxylierte und propyloxylierte Zucker eingesetzt werden. Beispielsweise sind Harnstoff, Guarnidin, Glycolsäure, Glycolate, Milchsäure, Lactate, Sucrose, Glucose, Fructose, Eruthrose, Erythritol, Sorbitol, Glycerin, Hexantriol, Propylenglycol, Butylenglycol, Hexylenglycol, alkoxylierte Glucose, Hyaluronsäure, Ethylenglykol, Ethylenglykolmonoethyl- oder -monobutylether, Propylenglykolmonomethyl, -monoethyl- oder -monobutylether, Diethylenglykolmonomethyl- oder -monoethylether vorteilhafte wasserlösliche Konditionierer.

Auch Polyarcylate (beispielsweise Carbopole der Typen 980, 981, 1382, 2984, 5984) können erfindungsgemäß vorteilhaft als wasserlösliche Konditionierer eingesetzt werden.

Ferner vorteilhaft sind Copolymere aus C₁₀₋₃₀-Alkylacrylaten und einem oder mehreren Monomeren der Acrylsäure, der Methacrylsäure oder deren Ester.

Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen. Ferner sind Verbindungen die die INCI-Bezeichnung Ammoniumacryloyldimethyltaurate/Vinylpyrrolidoncopolymere oder Acryloyldimethyltaurat tragen erfindungsgemäß vorteilhaft.

Weitere erfindungsgemäß vorteilhaft zu verwendende Konditionierer sind auch in Wasser lösliche oder dispergierbare anionische Polyurethane z. B. Polyurethan-1 und/oder Polyurethan-4.

Vorteilhaft im Sinne der vorliegenden Erfindung enthält die Zubereitung zum Zeitpunkt des Auftragens auf das Substrat ein oder mehrere Konditionierer in einer Gesamtkonzentration von 0,01 bis 1 Gewichts-%, bevorzugt von 0,05 bis 0,6 Gewichts-% und ganz besonders bevorzugt von 0,1 bis 0,3 Gewichts-%, jeweils bezogen auf das Gesamtgewicht der Zubereitung.

Erfindungsgemäß vorteilhaft beträgt die Viskosität der Zubereitung zum Zeitpunkt des Auftragens auf das Substrat kleiner 9000 mPas, bevorzugt kleiner 5000 mPas, besonders bevorzugt kleiner 3000 mPas.

Die Viskosität der Zubereitung läßt sich bei Bedarf durch den Zusatz geeigneter Mengen an Verdickern oder Gelbildnern erfindungsgemäß einstellen.

Erfindungsgemäß vorteilhaft beträgt das Gewichtsverhältnis von Substrat zu Zubereitung von 1:0,5 bis 1:5, wobei ein Gewichtsverhältnis von Substrat zu Zubereitung von 1:0,7 bis 1:3 erfindungsgemäß bevorzugt ist.

Das mit der erfindungsgemäßen Zubereitung imprägnierte Substrat kann eine feuchte oder trockene Anmutung aufweisen, daß heißt sich feucht oder trocken anfühlen. Da dieser sensorische Eindruck zu einem nicht unwesentlichen Teil auf den Gehalt an Wasser in der Zubereitung zurückzuführen ist, ist es erfindungsgemäß von Vorteil, wenn die Wasserkonzentration der Zubereitung auf dem Substrat unter 25 Gewichts-% bevorzugt unter 10 Gewichts-% und ganz besonders bevorzugt 5 Gewichts-% beträgt.

Erfindungsgemäß sind sowohl feuchte Substrate, die nach lmprägnierung einsatzfähig sind, als auch trockene Substrate, die nach der lmprägnierung mit der erfindungsgemäßen Zubereitung ganz oder teilweise getrocknet werden, oder bei denen die Zubereitung in pastöser oder fester Form auf das Substrat aufgetragen wird.

Erfindungsgemäß besonders bevorzugt sind trockene bzw. trocken anmutende Substrate, insbesondere trockene bzw. trocken anmutende Substrate aus Vlies, welche als Tücher dargereicht werden.

Erfindungsgemäß vorteilhaft ist die Verwendung des erfindungsgemäßen Substrates zur kosmetischen Reinigung und kosmetischen Pflege von Haut und Hautanhangsgebilden. Hierzu zählen erfindungsgemäß insbesondere die Haare und Nägel.

Erfindungsgemäß bevorzugt ist die Verwendung der erfindungsgemäßen Substrate zur Gesichtsreinigung und zum Entfernen von dekorativer Kosmetik (sogenanntes Abschminken) insbesondere zum Entfemen von Make-up.

Darüber hinaus eignen sich die erfindungsgemäßen Substrate hervorragend zur Reinigung von Gegenständen des täglichen Lebens (z.B. Geschirr, Tisch- und Schrankflächen, Autos).

Die Zahlenwerte in den Beispielen bedeuten Gewichtsprozente, bezogen auf das Gesamtgewicht der jeweiligen Zubereitungen.

### Beispiel 1

| O/W-Emulsion | Gewichts-% |
|---|---|
| Ceteareth-12 | 0,6 |
| Ceteareth-20 | 0,6 |
| Glycerylstearat | 0,3 |
| Cetylstearylalkohol | 0,2 |
| Paraffinum Liquidum | 8,0 |
| Ethylhexylstearat | 2,0 |
| Glycerin | 5,0 |
| Parfum | 0,5 |
| Panthenol | 0,25 |
| Natriumchlorid | 0,01 |
| Phenoxyethanol | 0,4 |
| Diazolidinylharnstoff | 0,25 |
| Zinkoxid 33 nm | 1,0 |
| Wasser | Ad 100 |

### Beispiel 2

| O/W-Emulsion | Gewichts-% |
|---|---|
| Ceteareth-12 | 0,6 |
| Ceteareth-20 | 0,6 |
| Glycerylstearat | 0,3 |
| Cetylstearylalkohol | 0,2 |
| Paraffinum Liquidum | 8,0 |
| Ethylhexylstearat | 2,0 |
| Glycerin | 5,0 |
| Bisabolol | 0,5 |
| Parfum | 0,5 |
| Phenoxyethanol | 0,4 |
| Diazolidinylharnstoff | 0,25 |
| Zinkoxid 33 nm | 0,3 |
| Wasser | Ad 100 |

### Beispiel 3

| O/W-Emulsion | Gewichts-% |
|---|---|
| Ceteareth-12 | 0,6 |
| Ceteareth-20 | 0,6 |
| Glycerylstearat | 1,5 |
| Cetylstearylalkohol | 2,0 |
| Paraffinum Liquidum | 6,0 |
| Ethylhexylstearat | 1,0 |
| Glycerin | 9,0 |
| Bisabolol | 0,1 |
| Parfum | 0,25 |
| Diazolidinylharnstoff | 0,15 |
| Zinkoxid 33 nm | 1,0 |
| Wasser | Ad 100 |

### Beispiel 4

| Hydrodispersion | Gewichts-% |
|---|---|
| Glycerin | 4,0 |
| Carbomer | 1,2 |
| Paraffinum Liquidum | 7,0 |
| Parabene | 1,0 |
| Natrium Hydroxid | 1,1 |
| Zinkoxid 33 nm | 1,5 |
| Parfum | 0,3 |
| Wasser | Ad 100 |

### Beispiel 5

| W/O-Emulsion | Gewichts-% |
|---|---|
| Glycerin | 3,0 |
| Paraffinum Liquidum | 20,0 |
| Polysorbate 65 | 0,9 |
| Cetyl PEG/PPG-10/1 Dimethicone | 2,2 |
| Cyclonmethicone | 13,0 |
| Polyglyceryl-2 Dipolyhydroxystearat | 1,4 |
| Tocopherol Acetate | 3,5 |
| Parabene | 0,3 |
| Zinkoxid 33 nm | 0,5 |
| Parfum | 0,3 |
| Wasser | Ad 100 |

### Beispiel 6

| Pickering O/W-Emulsion | Gewichts-% |
|---|---|
| Glycerin | 3,0 |
| Paraffinum Liquidum | 9,0 |
| Kalium Sorbat | 0,15 |
| Natrium Citrat | 0,2 |
| Hydroxyethylcellulose | 0,8 |
| Panthenol | 1,0 |
| Parabene | 0,3 |
| Phenoxyethanol | 0,3 |
| Simethicone | 0,15 |
| Titandioxid | 0,15 |
| Alumina | 0,15 |
| Zinkoxid 33 nm | 1,0 |
| Parfum | 0,3 |
| Wasser | Ad 100 |

### Beispiel 7

| O/W-Emulsion | Gewichts-% |
|---|---|
| Ceteareth-12 | 0,6 |
| Ceteareth-20 | 0,6 |
| Glycerylstearat | 0,3 |
| Cetylstearylalkohol | 0,2 |
| Paraffinum Liquidum | 8,0 |
| Ethylhexylstearat | 2,0 |
| Glycerin | 5,0 |
| Parfum | 0,5 |
| Panthenol | 0,25 |
| Natriumchlorid | 0,01 |
| Phenoxyethanol | 0,4 |
| Diazolidinylharnstoff | 0,25 |
| Zinkoxid 60 nm | 1,0 |
| Wasser | Ad 100 |

### Beispiel 8

| O/W-Emulsion | Gewichts-% |
|---|---|
| Ceteareth-12 | 0,6 |
| Ceteareth-20 | 0,6 |
| Glycerylstearat | 0,3 |
| Cetylstearylalkohol | 0,2 |
| Paraffinum Liquidum | 8,0 |
| Ethylhexylstearat | 2,0 |
| Glycerin | 5,0 |
| Bisabolol | 0,5 |
| Parfum | 0,5 |
| Phenoxyethanol | 0,4 |
| Diazolidinylharnstoff | 0,25 |
| Zinkoxid 60 nm | 0,3 |
| Wasser | Ad 100 |

### Beispiel 9

| O/W-Emulsion | Gewichts-% |
|---|---|
| Ceteareth-12 | 0,6 |
| Ceteareth-20 | 0,6 |
| Glycerylstearat | 1,5 |
| Cetylstearylalkohol | 2,0 |
| Paraffinum Liquidum | 6,0 |
| Ethylhexylstearat | 1,0 |
| Glycerin | 9,0 |
| Bisabolol | 0,1 |
| Parfum | 0,25 |
| Diazolidinylharnstoff | 0,15 |
| Zinkoxid 60 nm | 1,0 |
| Wasser | Ad 100 |

### Beispiel 10

| Hydrodispersion | Gewichts-% |
|---|---|
| Glycerin | 4,0 |
| Carbomer | 1,2 |
| Paraffinum Liquidum | 7,0 |
| Parabene | 1,0 |
| Natrium Hydroxid | 1,1 |
| Zinkoxid 60 nm | 1,5 |
| Parfum | 0,3 |
| Wasser | Ad 100 |

### Beispiel 11

| W/O-Emulsion | Gewichts-% |
|---|---|
| Glycerin | 3,0 |
| Paraffinum Liquidum | 20,0 |
| Polysorbate 65 | 0,9 |
| Cetyl PEG/PPG-10/1 Dimethicone | 2,2 |
| Cyclonmethicone | 13,0 |
| Polyglyceryl-2 Dipolyhydroxystearat | 1,4 |
| Tocopherol Acetate | 3,5 |
| Parabene | 0,3 |
| Zinkoxid 60 nm | 0,5 |
| Parfum | 0,3 |
| Wasser | Ad 100 |

### Beispiel 12

| Pickering O/W-Emulsion | Gewichts-% |
|---|---|
| Glycerin | 3,0 |
| Paraffinum Liquidum | 9,0 |
| Kalium Sorbat | 0,15 |
| Natrium Citrat | 0,2 |
| Hydroxyethylcellulose | 0,8 |
| Panthenol | 1,0 |
| Parabene | 0,3 |
| Phenoxyethanol | 0,3 |
| Simethicone | 0,15 |
| Titandioxid | 0,15 |
| Alumina | 0,15 |
| Zinkoxid 60 nm | 1,0 |
| Parfum | 0,3 |
| Wasser | Ad 100 |

## Patentansprüche

1. Kosmetische Produkte umfassend ein Substrat, welches imprägniert ist mit einer kosmetischen und/oder dermatologischen Zubereitung enthaltend
a) Wasser
b) Öl
c) einen oder mehrere Emulgatoren
d) Zinkoxid einer Partikelgröße, die aus dem Bereich von 20 bis 100 nm gewählt wird, und wobei das Substrat gewählt wird aus der Gruppe der Tücher mit folgenden Parametern
| einer Reißkraft von | | |
|---|---|---|
| | | [N/50mm] |
| im trockenen Zustand | Maschinenrichtung | >60 |
| | Querrichtung | >20 |
| im getränkten Zustand | Maschinenrichtung | >4 |
| | Querrichtung | >10 |
| einer Dehnfähigkeit von | | |
|---|---|---|
| im trockenen Zustand | Maschinenrichtung | 15 % bis 100 % |
| | Querrichtung | 40 % bis 120 % |
| im getränkten Zustand | Maschinenrichtung | 15 % bis 100 % |
| | Querrichtung | 40 % bis cx120 % |

2. Kosmetische Produkte nach Anspruch 1, bei denen das Substrat gewählt wird aus der Gruppe der Tücher mit folgenden Parametern:
| eine Reißkraft von | | |
|---|---|---|
| | | [N/50mm] |
| im trockenen Zustand | Maschinenrichtung | >80 |
| | Querrichtung | >30 |
| im getränkten Zustand | Maschinenrichtung | >60 |
| | Querrichtung | >20 |
| einer Dehnfähigkeit von | | |
|---|---|---|
| im trockenen Zustand | Maschinenrichtung | 20 % bis 50 % |
| | Querrichtung | 50 % bis 85 % |
| im getränkten Zustand | Maschinenrichtung | 20 % bis 40 % |
| | Querrichtung | 50 % bis 85 % |

3. Produkte nach Anspruch 1, **dadurch gekennzeichnet, daß** es sich bei dem Substrat um ein Tuch aus Vließ handelt.

4. Produkte nach einem der Ansprüche 1 bis 3, **dadurch gekennzeichnet, daß** die Zubereitung zum Zeitpunkt des Auftragens auf das Substrat Zinkoxid in einer Gesamtkonzentration von 0,05 bis 20 Gewichts-% bezogen auf das Gesamtgewicht der Zubereitung enthält.

5. Produkte nach einem der vorhergehenden Ansprüche als feucht oder trocken anmutende Tücher.

6. Verwendung von Produkten nach einem der Ansprüche 1 bis 4 zur kosmetischen Reinigung und kosmetischen Pflege von Haut und Hautanhangsgebilden.

## Claims

1. Cosmetic products comprising a substrate which is impregnated with a cosmetic and/or dermatological preparation comprising
a) water
b) oil
c) one or more emulsifiers
d) zinc oxide of a particle size which is selected from the range from 20 to 100 nm, and where the substrate is selected from the group of tissues with the following parameters:
| a tear strength of | | |
|---|---|---|
| | | [N/50 mm] |
| in the dry state | machine direction | >60 |
| | transverse | >20 |
| | direction | |
| in the impregnated state | machine direction | >4 |
| | transverse direction | >10 |
| an expandability of | | |
|---|---|---|
| in the dry state | machine direction | 15% to 100% |
| | transverse direction | 40% to 120% |
| in the impregnated state | machine direction | 15% to 100% |
| | transverse direction | 40% to 120% |

2. Cosmetic products according to Claim 1 in which the substrate is selected from the group of tissues with the following parameters:
| a tear strength of | | |
|---|---|---|
| | | [N/50 mm] |
| in the dry state | machine direction | >80 |
| | transverse direction | >30 |
| in the impregnated state | machine direction | >60 |
| | transverse direction | >20 |
| an expandability of | | |
|---|---|---|
| in the dry state | machine direction | 20% to 50% |
| | transverse direction | 50% to 85% |
| in the impregnated state | machine direction | 20% to 40% |
| | transverse direction | 50% to 85% |

3. Products according to Claim 1, **characterized in that** the substrate is a tissue made of nonwoven.

4. Products according to one of Claims 1 to 3, **characterized in that** the preparation at the point of application to the substrate comprises zinc oxide in a total concentration of from 0.05 to 20% by weight, based on the total weight of the preparation.

5. Products according to one of the preceding claims as tissues that seem wet or dry.

6. Use of products according to one of Claims 1 to 4 for cosmetic cleansing and cosmetic care of skin and skin appendages.

## Revendications

1. Produits cosmétiques comprenant un substrat qui est imprégné d'une préparation cosmétique et/ou dermatologique, contenant
a) de l'eau
b) de l'huile
c) un ou plusieurs émulsifiants
d) de l'oxyde de zinc d'une grosseur de particules choisie dans la plage de 20 à 100 nm,
et où le substrat est choisi dans le groupe des lingettes présentant les paramètres suivants :
| une résistance à la déchirure de | | |
|---|---|---|
| | | [N/50mm] |
| à l'état sec | sens de la machine | > 60 |
| | sens transversal | > 20 |
| à l'état imbibé | sens de la machine | > 4 |
| | sens transversal | > 10 |
| une aptitude à l'allongement de | | |
|---|---|---|
| à l'état sec | sens de la machine | 15% à 100% |
| | sens transversal | 40% à 120% |
| à l'état imbibé | sens de la machine | 15% à 100% |
| | sens transversal | 40% à 120% |

2. Produits cosmétiques selon la revendication 1, dans lesquels le substrat est choisi dans le groupe des lingettes présentant les paramètres suivants :
| une résistance à la déchirure de | | |
|---|---|---|
| | | [N/50mm] |
| à l'état sec | sens de la machine | > 80 |
| | sens transversal | > 30 |
| à l'état imbibé | sens de la machine | > 60 |
| | sens transversal | > 20 |
| une aptitude à l'allongement de | | |
|---|---|---|
| à l'état sec | sens de la machine | 20% à 50% |
| | sens transversal | 50% à 85% |
| à l'état imbibé | sens de la machine | 20% à 40% |
| | sens transversal | 50% à 85% |

3. Produits selon la revendication 1, **caractérisés en ce qu'**il s'agit, pour le substrat, d'une lingette en non-tissé.

4. Produits selon l'une quelconque des revendications 1 à 3, **caractérisés en ce que** la préparation contient au moment de l'application sur le substrat de l'oxyde de zinc en une concentration totale de 0,05 à 20% en poids, par rapport au poids total de la préparation.

5. Produits selon l'une quelconque des revendications précédentes comme lingettes qui semblent humides ou sèches.

6. Utilisation de produits selon l'une quelconque des revendications 1 à 4 pour le nettoyage cosmétique et le soin cosmétique de la peau et des phanères.
